# EUROPEAN PATENT SPECIFICATION

(11) **EP 3 105 200 B1**
(45) Date of publication and mention of the grant of the patent: **18.04.2018**
(21) Application number: 15705949.4
(22) Date of filing: 11.02.2015
(51) Int. Cl.: C07C 5/32, C07C 5/333, C07C 5/42, C07C 15/46

(54) **METHOD FOR OPERATING A DEHYDROGENATION REACTOR FOR THE DEHYDROGENATION OF HYDRO-CARBONS**
VERFAHREN ZUM BETRIEB EINES DEHYDRIERUNGSREAKTORS ZUR DEHYDRIERUNG VON KOHLENWASSERSTOFFEN
PROCÉDÉ PERMETTANT DE FAIRE FONCTIONNER UN RÉACTEUR DE DÉSHYDROGÉNATION POUR LA DÉSHYDROGÉNATION D'HYDROCARBURES

(30) Priority: 12.02.2014 EP 14154816
(43) Date of publication of application: 21.12.2016
(73) Proprietor: INEOS Styrolution Group GmbH, 60325 Frankfurt am Main (DE)
(72) Inventor: MILLER, Michael L., League City, Texas 77573 (US); BROWN, Timothy A., League City, Texas 77573 (US); SHAFER, Clay, League City, Texas 77573 (US)
(74) Representative: Jacobi, Markus Alexander
(86) International application number: PCT/EP2015/052857
(87) International publication number: WO 2015/121297

(56) References cited:
- US-A- 2 428 624
- US-A- 3 505 422
- US-A1- 2005 080 306

## Description

The invention relates to a method for operating a dehydrogenation reactor for the dehydrogenation of hydrocarbons, wherein the dehydrogenation reactor comprises a potassium promoted iron oxide catalyst and the hydrocarbons are dehydrogenated in contact with the catalyst.

Styrene, which is used in large amounts in the production of synthetic rubbers, ABS resins and polystyrene, is often manufactured by the dehydrogenation of ethylbenzene. The dehydrogenation process is carried out on industrial scale in large dehydrogenation reactors. A dehydrogenation reactor typically comprises at least one dehydrogenation reactor vessel, having a reactor inlet for receiving a feed and a reactor outlet for discharging a reactor effluent. The reactor vessel generally comprises catalyst particles.
In the process of making styrene, ethylbenzene with excess steam is dehydrogenated in contact with the catalyst. The most common catalyst is iron oxide based and comprises potassium and other promoters.
Such a catalyst is disclosed in US 6,242,379. The catalyst comprises from about 40 to about 90 % by weight iron oxide (Fe₂O₃), from about 5 to about 20 % by weight of an alkali metal compound (calculated as an alkali metal oxide), from about 1 ppm to about 1000 ppm of a source of palladium or platinum, from about 0.5 to about 10 % by weight of a molybdenum or tungsten compound and from about 4 to about 12 % by weight of a cerium compound. The alkali metal compound is most preferably potassium carbonate (K₂CO₃).

During the operation of a dehydrogenation reactor, comprising such a catalyst, the alkali metal compound (K₂CO₃) can convert into different forms, such as potassium oxide (K₂O), potassium hydroxide (KOH), K₂Fe₂O₄, K₂Fe₂₂O₃₄ and other potassium ferrites. The temperatures near the inlet of the reactor are hot enough to vaporize significant amounts of KOH, which re-condenses in cooler portions of the catalyst bed. Potassium species migrate from the hotter inlet area of the catalyst bed to cooler portions of the bed closer to the outlet. The temperature in the reactor is also sufficient to generate carbon dioxide (CO₂) during the dehydrogenation of hydrocarbons, which causes the KOH to revert to non-volatile K₂CO₃. The re-deposited KOH and K₂CO₃ bind catalyst particles together.

As time passes, the re-deposited potassium species accumulate. The pressure drop increases in the catalyst bed and the reaction is impeded and effectively eliminated in the fused, agglomerated particles. The catalyst bed has to be exchanged. Typically, the used catalyst is vacuumed from the cooled reactor.

When dehydrogenation catalysts are unloaded from the reactor after typical operating lives of 2 to 5 years or more, the used catalyst typically has few if any agglomerates in the upper portion of the catalyst beds. As unloading continues, increasing amounts of agglomerates are found making it difficult to vacuum catalyst from the reactor. If agglomeration is severe enough, the fused agglomerates must be broken into manageabe chunks first. It is therefore desirable to operate the dehydrogenation reactor such that agglomeration of the catalyst is reduced.

From US 2005/0080306 a method for cooling down the reactor catalyst is known wherein contacting of the dehydrogenation feed with the dehydrogenation catalyst is terminated followed by the contacting of the dehydrogenation catalyst with a carbon dioxide-containing cooling gas. When the temperature of the catalyst has been reduced, contacting of the dehydrogenation catalyst with the carbon-dioxide comprising cooling gas is terminated. In another embodiment the method comprises two cooling steps after terminating contacting the dehydrogenation feed with the dehydrogenation catalyst. In a first cooling step, the dehydrogenation catalyst is contacted with a first cooling gas, comprising steam for a first period of time. Thereafter, the contacting of the dehydrogenation catalyst with the first cooling gas is terminated and, in a second cooling step, the dehydrogenation catalyst is then contacted with a second cooling gas, comprising a major portion carbon dioxide. After the shutdown, the dehydrogenation catalyst is removed.

According to US 2005/0080306 it has been found that if steam is used to cool the temperature of the dehydrogenation catalyst below a certain level, various reactions can occur with the dehydrogenation catalyst bed that cause unwanted agglomeration. The potassium compounds in the catalyst can form potassium hydroxide (KOH) and iron oxide. Further, the iron oxide can hydrate to FeOOH which, in combination with potassium hydroxide, which is liquid above 360°C, forms a sticky mixture which binds catalyst particles together. Thus, steam should be avoided for cooling the catalyst below a certain level. Further, it has been found that a high carbon dioxide concentration in the cooling gas is desirable due to the high heat capacity of carbon dioxide. Additionally, the use of carbon dioxide can lead to the formation of potassium carbonate in the catalyst bed instead of potassium hydroxide and potassium oxide which are associated with undesirable catalyst agglomeration.

The known methods for operating a dehydrogenation reactor with a potassium promoted iron oxide catalyst comprising potassium can reduce agglomeration of catalyst particles during shut-down of the reactor, but do not address agglomeration during other operations, such as start-up or steaming procedures. Steaming procedures expose the catalyst to steam only and are performed when needed to remove excess coke from the catalyst bed that can reduce catalyst activity.

It is one object of the present invention to provide an improved method for operating a dehydrogenation reactor during start-up, shut-down and steaming procedures with which catalyst agglomeration can be reduced.

Accordingly a method for operating a dehydrogenation reactor for the dehydrogenation of hydrocarbons is provided, wherein the dehydrogenation reactor comprises a potassium promoted iron oxide catalyst, the hydrocarbons being dehydrogenated in contact with the catalyst and carbon dioxide is introduced during:
a)start-up of the reactor,
b)shut-down of the reactor, and
c)steaming procedures,
wherein the carbon dioxide is introduced in conjunction with steam in at least one method step.

The dehydrogenation reactor comprises at least one reactor vessel having a reactor inlet for receiving a feed and a reactor outlet for discharging a reactor effluent. If the system comprises more than one reactor vessel, the vessels can be arranged in parallel, in series or both. The catalyst is located inside the reactor vessel in form of a catalyst bed.

Typically the potassium is included in the catalyst in form of K₂CO₃. It is mulled with iron oxides and various promoters then calcinated at temperatures significantly higher than dehydrogenation reaction conditions. Some of the K₂CO₃ decomposes to K₂O, some also forms various potassium ferrites.

During operation of the dehydrogenation reactor and depending on the conditions the catalyst comprises several potassium species such as potassium oxide (K₂O), potassium hydroxide (KOH), potassium carbonate (K₂CO₃), K₂Fe₂O₄, K₂Fe₂₂O₃₄ and other potassium ferrites.

Start-up, shut-down and steaming are critical operations, as the temperature of the catalyst bed is high enough for KOH to evaporate and migrate to cooler portions of the catalyst bed, but the catalyst does not generate CO₂. CO₂ can convert the volatile KOH to K₂CO₃ which has a very low volatility and does not melt until temperatures reach 891 °C (1635.8 F). Additionally, KOH melts at 360°C (682 F) and gravity probably pulls some of the liquid KOH downward.

Under reaction conditions, the dehydrogenation catalysts generate some CO₂ while hydrocarbons are being dehydrogenated in contact with the catalyst. The dehydrogenation catalyst generate CO₂ from a balance of coking reactions that continue the dehydrogenation process to higher degrees, and gasification reactions that convert the coke to CO and subsequently to CO₂ through the water gas shift reaction. The generated CO₂ stabilizes the catalyst bed by converting volatile KOH to stable K₂CO₃. CO₂ concentrations increase as the flow progresses through the catalyst bed or through multiple reactor vessels. Equilibrium strongly favors K₂CO₃ when even small concentrations of CO₂ are present.

In the presence of water or steam in absence of CO₂, K₂CO₃ and KO revert to KOH. During startups and shutdowns, the catalyst bed temperatures are not high enough to generate significant concentrations of CO₂, but temperatures are high enough to convert K₂CO₃ back to KOH, to melt KOH, and to vaporize some KOH.

In order to stabilize the catalyst during start-up, shut-down or steaming, where little or no CO₂ is generated, CO₂ is introduced into the reactor. In case of steaming or shut-down of the reactor, the introduction of CO₂ is performed in conjunction with steam for at least a certain fraction of time.

In one embodiment of the invention, CO₂ which has passed through the reactor is being recirculated. The CO₂ is collected after the reactor, fed through a compressor and fed back into the reactor in order to minimize the amount of CO₂ required in the process.

For the start-up of the reactor according to alternative a), the method comprises the following steps:
i. heating the reactor to a first temperature by a recirculating gas stream comprising nitrogen,
ii. heating the reactor to a second temperature by introducing steam, and optionally continuing nitrogen recirculation along with the steam,
iii. optionally, checking for leaks, and
iv. heating the reactor to a third temperature and stopping carbon dioxide introduction before introducing the hydrocarbon,
wherein the carbon dioxide is introduced in step i) via the recirculating nitrogen gas stream and in steps ii) and iii) via the steam and optional recirculating nitrogen.

In the first step i) the reactor is heated by a recirculating gas stream to the first temperature. The gas stream comprises nitrogen and carbon dioxide. The gas stream passes through a steam superheating furnace where it is heated, through the catalyst beds, through a reactor effluent heat exchanger and through condensing exchangers. Then the gas stream is passed to compressors which force the stream back to the furnace. Additional nitrogen or carbon dioxide can be added to the gas stream as required. The gas is kept recirculating through the system until the first temperature is reached. During this step, any air inside the apparatus is displaced by the introduced nitrogen gas and optionally a leak check can be performed.

In step ii) of the method the gas stream is switched to steam and the nitrogen gas flow is terminated. CO₂ is mixed to the steam before the gas is passed through the furnace. The gas flows until the second temperature is reached. Steam has a higher heat capacity than nitrogen so that heating is accelerated compared to using nitrogen only.

In a variant of the method the nitrogen recirculation is not terminated but continued along with the introduction of steam. The steam condenses in the condensing exchanges and the nitrogen and carbon dioxide are passed back to the furnace through the compressor. The amount of nitrogen recirculation can be reduced compared to the amount used in step i). In this variant the amount of CO₂ which has to be added to the steam is reduced as most of the introduced CO₂ recirculates together with the nitrogen.

In the optional step iii) the reactor is checked for leaks. Typically the reactor is heated to a temperature of about 540°C (1000 F) for the leak test. If no leaks are found, the start-up is continued.

In step iv) the reactor is heated to the third temperature using steam as rapidly as possible. Typically, for industrial scale reactors, the rate is less than 55 °C (100 F) per hour. When the third temperature is reached, the introduction of CO₂ is stopped and the hydrocarbon is introduced and the temperature is adjusted to the desired value for optimal dehydrogenation. Preferably the introduction of CO₂ is stopped just before the hydrocarbons are introduced.

For the shut-down of the reactor according to alternative b), the method comprises the following steps:
i. stopping the introduction of hydrocarbons into the reactor, the reactor being at a third temperature,
ii. cooling the reactor to a first temperature using a coolant gas comprising steam and optionally adding nitrogen and recirculating the nitrogen along with the steam,
iii. cooling the reactor to a fourth temperature using a coolant gas comprising nitrogen,
wherein the carbon dioxide is introduced in step ii) via the coolant gas comprising steam and optional recirculating nitrogen and in step iii) via the coolant gas comprising nitrogen.

In the first step i) the introduction of hydrocarbons into the reactor is stopped. The temperature of the reactor is at or near the temperature used during the dehydrogenation reaction.

In the second step ii) the reactor is cooled using a cooling gas comprising steam. CO₂ is mixed to the steam before the gas is passed through the reactor. The gas flows until the first temperature is reached.

In a variant of the method the cooling gas also comprises nitrogen which is being recirculated. The steam condenses in a condensing heat exchanger and the nitrogen and carbon dioxide is passed back to the reactor through the compressor. In this variant the amount of CO₂ which has to be mixed to the steam is reduced as most of the introduced CO₂ recirculates together with the nitrogen.

In the third step iii) the introduction of steam is stopped and a cooling gas comprising nitrogen is passed through the reactor. CO₂ is mixed to the cooling gas stream as required. The cooling gas recirculates though the compressor back to the reactor. The gas circulation is continued until the reactor has reached the fourth temperature.

After the shut-down is completed, maintenance such as removal of the catalyst bed can be performed.

For steaming procedures according to alternative c), the method comprises the following steps beginning from normal operation:
i. stopping the introduction of hydrocarbons into the reactor, the reactor being at a third temperature
ii. adjusting the reactor to and holding at a fifth temperature with steam and optionally adding nitrogen and recirculating the nitrogen,
iii. adjusting the temperature to a third temperature and stopping the introduction of carbon dioxide before introducing the hydrocarbon,
wherein the carbon dioxide is introduced in steps ii) and iii) via the steam and optional recirculating nitrogen.

In the first step i) the introduction of hydrocarbons into the reactor is stopped. The temperature of the reactor is at or near the temperature used during the dehydrogenation reaction.

In the second step ii) the temperature of the reactor is set to the fifth temperature, the steaming temperature. Steam and CO₂ is passed through a steam superheating furnace and then introduced into the reactor. After passing through the reactor the steam condenses in a condensing exchanger.

In a variant of the invention nitrogen which is being recirculated is mixed with the steam. The steam condenses in the condensing heat exchanger and the nitrogen and carbon dioxide is passed back to the reactor through the compressor. In this variant the amount of CO₂ which has to be added to the steam is reduced as most of the introduced CO₂ recirculates together with the nitrogen.

In final step iii) the temperature is adjusted to the third temperature using the steam and/or nitrogen gas flowing through the reactor. CO₂ is still introduced with the gas stream until the third temperature is reached. The introduction of CO₂ is then stopped just before the hydrocarbons are introduced.

It is also possible to include steaming procedures into the start-up sequence. In this case, the method comprises the following steps:
i. heating the reactor to a first temperature by a recirculating gas stream comprising nitrogen,
ii. heating to and holding at a fifth temperature with steam and optionally recirculating the nitrogen,
iii. adjusting the temperature to a third temperature and stopping the introduction of carbon dioxide before introducing the hydrocarbon,
wherein the carbon dioxide is introduced in step i) via the recirculating nitrogen gas stream and in steps ii) and iii) via the steam and optional recirculating nitrogen.

In the first step i) the reactor is heated by a recirculating gas stream to the first temperature.

The gas stream comprises nitrogen and carbon dioxide. The gas stream passes through the steam superheating furnace where it is heated, through the catalyst beds, through the reactor effluent heat exchanger and through condensing exchangers. Then the gas stream is passed to compressors which force the stream back to the furnace. Additional nitrogen or carbon dioxide can be added to the gas stream as required. The gas is kept recirculating through the system until the first temperature is reached.

In the second step ii) steam mixed with CO₂ is passed through the steam superheating furnace and then introduced into the reactor to heat the reactor to the fifth temperature. After passing though the reactor, the steam condenses in the condensing exchangers.

In a variant of the invention nitrogen which is being recirculated is mixed with the steam. The steam condenses in the condensing heat exchanger and the nitrogen and carbon dioxide is passed back to the reactor through the compressor. In this variant the amount of CO₂ which has to be added to the steam is reduced as most of the introduced CO₂ recirculates together with the nitrogen.

During steaming procedures the reactor is held at the fifth temperature for a time of 30 minutes to 4 hours. During this time excess coke is removed from the catalyst beds.

The first temperature used in the operating method is the transition temperature for going from nitrogen to steam during start-up and from steam to nitrogen during shut-down respectively. The first temperature is preferably in the range of from 260 °C (500 F) to 370°C (700 F).
During start-up, a leak check can be performed. The leak check is preferably performed with the reactor being at the second temperature. Preferably the second temperature is in the range of from 480 °C (900 F) to 590 °C (1100 F).
Hydrocarbons are introduced into the reactor when the reactor is at the third temperature. Preferably the third temperature is in the range of from 540 °C (1000 F) to 650 °C (1200 F).

When a shut-down is performed, the reactor is cooled down to the fourth temperature where the catalyst can be safely handled. Preferably the fourth temperature is in the range of from 20 °C (68 F) to 50 °C (122 F).

Steaming procedures are performed with the reactor being held at the fifth temperature. Preferably the fifth temperature is in the range of from 540 °C (1000 F) to 650 °C (1200 F).

The amount of carbon dioxide (CO₂) which is added to the recirculating nitrogen gas is a concentration of from 2 to 50% by volume. The % by volume is given with respect to the entire gas stream.
When carbon dioxide is added to the introduced steam, the concentration of carbon dioxide in the steam is from 0.1 to 20 % by volume. Especially preferred is a concentration of carbon dioxide in the steam of from 0.2 to 2 % by volume.

In the invention the amount of introduced carbon dioxide is chosen such that the concentration of carbon dioxide at the catalyst during a) start-up, b) shut-down or c) steaming procedures is between 0.1 to 20 % by volume.
In one embodiment of the invention the catalyst is an iron based dehydrogenation catalyst comprising from 40 to 90 % by weight iron and from 5 to 30 % by weight potassium.

The hydrocarbon which is introduced into the reactor is preferably selected from the group of alkylbenzenes such as ethylbenzene, methylethylbenzenes, diethylbenzene and alkanes such as propane, butane and linear alkanes through C9 to C15.

In further embodiments of the invention a vent gas analyzer is used to monitor CO₂ concentrations in the reactor effluent. The amount of introduced CO₂ can then be adjusted so that the CO₂ concentration in the vent gas is within a predetermined interval.

An embodiment of the present invention will now be described further, by way of example, with reference to the accompanying drawing, in which:

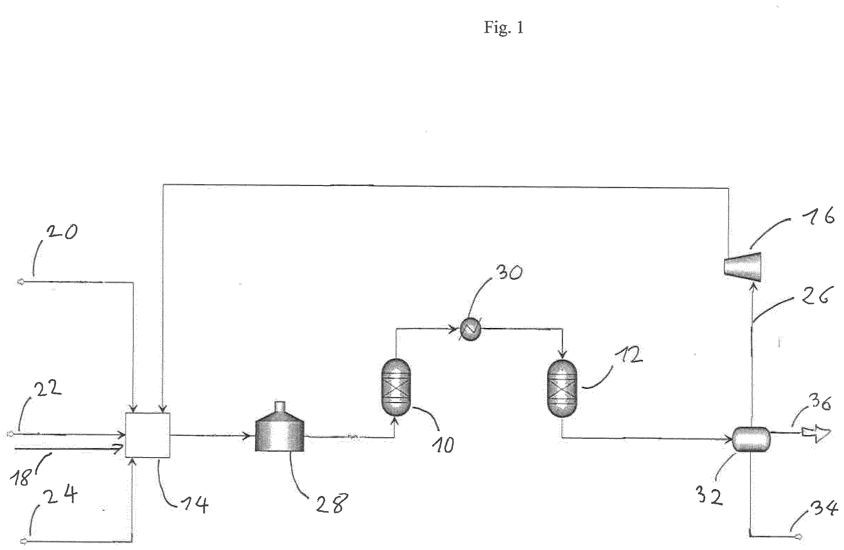

Figure 1 shows a schematic flow diagram depicting the steam, nitrogen and carbon dioxide flow through a system comprising two reactors.

The gas flow through a system comprising two reactor vessels is schematically depicted in figure 1. Steam can be introduced into the system by a steam source line 20, nitrogen by a nitrogen source line 22 and carbon dioxide can be introduced by a carbon dioxide source line 24. The gases are mixed using a mixer 14 before the gas is led into a heater 28. The heater may be a steam superheating furnace. The heated gas is then fed into the first reactor vessel 10. In the embodiment shown in figure 1, the effluent of the first reactor vessel 10 is first led through a re-heating device 30 before the gas stream is introduced into the second reactor vessel 12.

The effluent is then passed to a condensing heat exchanger 32. Water is condensed inside the condensing heat exchanger 32 and leaves the system through line 34. Nitrogen and carbon dioxide are led to a compressor 16 through recirculation line 26 and are then fed back into the mixer 14.

In normal dehydrogenation operation, hydrocarbons can be introduced into the system via a hydrocarbon source line 18 and are removed through line 36 after the effluent has passed through the heat exchanger 32.

In further embodiments of the invention, different configurations are possible, e.g. a different number of reactor vessels may be used or several reactor vessels could be used in a parallel configuration.

The following example, the figure and the claims further illustrate the invention.

### Example 1:

During the start-up of the reactor, air is purged from the equipment with nitrogen, then nitrogen and carbon dioxide are introduced. To make up for losses to flare, nitrogen and carbon dioxide continue to be added so that flow rates of about 27,215.54 kg/h (60,000 lb/hr) including the recycled nitrogen and carbon dioxide can be maintained through the reactors while heating to 315.5°C (600 F). After passing through heat exchangers, a compressor recirculates the gas back to the mixers. The CO₂ concentration in the reactor effluent is 10 vol%.

After reaching the first temperature of 315.5°C (600 F), steam is introduced into the mixer along with recirculated nitrogen and carbon dioxide while heating to 537.8°C (1000 F). The total flow rates to the reactors are 45,359.23 kg/h (100,000 lb/hr) steam, 6,912.74kg/h (15,240 lb/hr) of nitrogen, and 494.87 kg/h (1,091 lb/hr) of carbon dioxide. Through the recirculation line, 6,803.88 kg/h (15,000 lb/hr) of nitrogen and 453.59 kg/h (1,000 lb/hr) of carbon dioxide are fed back into the mixer. About 108.86 kg/h (240 lb/hr) nitrogen and 82.55 kg/h (182 lb/hr) of carbon dioxide are also added to the mixer to make up for losses to condensate and flare. The reactor is heated further. When the third temperature is reached, the introduction of carbon dioxide is stopped. The reactor can then be used for the dehydrogenation of hydrocarbons.

## Claims

1. Method for operating a dehydrogenation reactor for the dehydrogenation of hydrocarbons wherein the dehydrogenation reactor comprises a potassium promoted iron oxide catalyst the hydrocarbons being dehydrogenated in contact with the catalyst, **characterized in that** carbon dioxide is introduced during
a) start-up of the reactor,
b) shut-down of the reactor, and
c) steaming procedures,
**characterized in that**
- the method comprises the following steps during start-up (a):
i. heating the reactor to a first temperature by a recirculating gas stream comprising nitrogen,
ii. heating the reactor to a second temperature by introducing steam, and optionally continuing nitrogen recirculation along with the steam,
iii. optionally, checking for leaks, and
iv. heating the reactor to a third temperature and stopping carbon dioxide introduction before introducing the hydrocarbon,
wherein the carbon dioxide is introduced in step i) via the recirculating nitrogen gas stream and in steps ii) and iv) via the steam and optional recirculating nitrogen,
- the method comprises the following steps during shut-down (b):
i. stopping the introduction of hydrocarbons into the reactor, the reactor being at a third temperature,
ii. cooling the reactor to a first temperature using a coolant gas comprising steam and optionally adding nitrogen and recirculating the nitrogen along with the steam,
iii. cooling the reactor to a fourth temperature using a recirculating coolant gas comprising nitrogen,
wherein the carbon dioxide is introduced in step ii) via the coolant gas comprising steam and optional recirculating nitrogen and in step iii) via the coolant gas comprising nitrogen,
- the method comprises the following steps during steaming procedures (c) beginning from normal operation:
i. stopping the introduction of hydrocarbons into the reactor, the reactor being at a third temperature
ii. adjusting the reactor to and holding at a fifth temperature with steam and optionally adding nitrogen and recirculating the nitrogen,
iii. adjusting the temperature to a third temperature and stopping the introduction of carbon dioxide before introducing the hydrocarbon,
wherein the carbon dioxide is introduced in steps ii) and iii) via the steam and optional recirculating nitrogen,
wherein the concentration of carbon dioxide in the steam is from 0.1 to 20 % by volume and the concentration of carbon dioxide in the nitrogen gas is from 2 to 50% by volume.

2. The method of claim 1, **characterized in that** carbon dioxide recirculation is practiced.

3. The method of claim 1 or 2, **characterized in that** steaming-procedures are incorporated into a start-up comprising the following steps:
i. heating the reactor to a first temperature by a recirculating gas stream comprising nitrogen,
ii. heating to and holding at a fifth temperature with steam and optionally recirculating the nitrogen,
iii. adjusting the temperature to a third temperature and stopping the introduction of carbon dioxide before introducing the hydrocarbon,
wherein the carbon dioxide is introduced in step i) via the recirculating nitrogen gas stream and in steps ii) and iii) via the steam and optional recirculating nitrogen.

4. The method of one of claims 1 or 2, **characterized in that** the fifth temperature is maintained for a time of 30 minutes to 4 hours.

5. The method of claim 1 or 2, **characterized in that** the first temperature is in the range of from 260 °C to 370°C.

6. The method of claim 1 or 2, **characterized in that** the second temperature is in the range of from 480 °C to 590 °C.

7. The method of one of claims 1 or 2, **characterized in that** the third temperature is in the range of from 540 °C to 650 °C.

8. The method of claim 1 or 2, **characterized in that** the fourth temperature is in the range of from 20 °C to 50 °C.

9. The method of claim 1 or 2, **characterized in that** the fifth temperature is in the range of from 540 °C to 650 °C.

10. The Method of any one of claims 1 to 9, **characterized in that** the catalyst is an iron based dehydrogenation catalyst comprising from 40 to 90 % by weight iron and from 5 to 30 % by weight potassium.

11. The method of any one of claims 1 to 10, **characterized in that** the hydrocarbon is selected from the group of alkylbenzenes such as ethylbenzene, methylethylbenzenes, diethylbenzene and alkanes such as propane, butane and linear alkanes through C9 to C15.

## Patentansprüche

1. Verfahren zum Betreiben eines Dehydrierungsreaktors zur Dehydrierung von Kohlenwasserstoffen, wobei der Dehydrierungsreaktor einen Kaliumunterstützten Eisenoxidkatalysator umfasst, wobei die Kohlenwasserstoffe in Kontakt mit dem Katalysator dehydriert werden, **dadurch gekennzeichnet, dass** Kohlendioxid eingeleitet wird während
a. Hochfahren des Reaktors
b. Herunterfahren des Reaktors, und
c. Dämpfverfahren,
**dadurch gekennzeichnet, dass**
- das Verfahren die folgenden Schritte während des Hochfahrens (a) umfasst:
i. Heizen des Reaktors auf eine erste Temperatur durch einen rezirkulierenden Gasstrom umfassen Stickstoff,
ii. Heizen des Reaktors auf eine zweite Temperatur durch Einleiten von Dampf und optional Fortsetzen der Stickstoffrezirkulation zusammen mit dem Dampf,
iii. optional Überprüfung auf Lecks, und
iv. Heizen des Reaktors auf eine dritte Temperatur und Stoppen der Kohlendioxideinleitung vor Einleitung des Kohlenwasserstoffs,
wobei das Kohlendioxid in Schritt i) eingeleitet wird über den rezirkulierenden Sickstoffgasstrom und in den Schritten ii) und iv) über den Dampf und optionalen rezirkulierenden Stickstoff,
- das Verfahren die folgenden Schritte während des Herunterfahrens (b) umfasst:
i. Stoppen der Einleitung von Kohlenwasserstoffen in den Reaktor, wobei der Reaktor auf einer dritten Temperatur ist,
ii. Kühlen des Reaktors auf eine erste Temperatur unter Verwendung eines Kühlgases umfassend Dampf und optional Hinzufügen von Stickstoff und Rezirkulieren des Stickstoffs zusammen mit dem Dampf,
iii. Kühlen des Reaktors auf eine vierte Temperatur unter Verwendung eines rezirkulierenden Kühlgases umfassend Stickstoff,
wobei das Kohlendioxid in Schritt ii) eingeleitet wird über das Kühlgas umfassend Dampf und optionalen rezirkulierenden Stickstoff und in Schritt iii) über das Kühlgas umfassend Stickstoff,
- das Verfahren die folgenden Schritte während der Dämpfverfahren (c) umfasst ausgehend von normalem Betrieb:
i. Stoppen der Einleitung von Kohlenwasserstoffen in den Reaktor, wobei der Reaktor auf einer dritten Temperatur ist,
ii. Einstellen des Reaktors auf und Halten bei einer fünften Temperatur mit Dampf und optional Hinzufügen von Stickstoff und Rezirkulieren des Stickstoffs,
iii. Einstellen der Temperatur auf eine dritte Temperatur und Stoppen der Einleitung von Kohlendioxid vor dem Einleiten des Kohlenwasserstoffs,
wobei das Kohlendioxid in den Schritten ii) und iii) über den Dampf und optionalen rezirkulierenden Stickstoff eingeleitet wird,
wobei die Konzentration von Kohlendioxid in dem Dampf von 0,1 bis 20 vol. % beträgt und die Konzentration von Kohlendioxid in dem Strickstoffgas von 2 bis 50 vol. % beträgt.

2. Das Verfahren nach Anspruch 1, **dadurch gekennzeichnet, dass** Kohlendioxidrezirkulation genutzt wird.

3. Das Verfahren nach Anspruch 1 oder 2, **dadurch gekennzeichnet, dass** Dämpfverfahren in ein Hochfahren umfassend folgende Schritte eingebunden sind:
i. Heizen des Reaktors auf eine erste Temperatur durch einen rezirkulierenden Gasstrom umfassend Stickstoff,
ii. Heizen auf und Halten bei einer fünften Temperatur mit Dampf und optional Rezirkulieren des Stickstoffs,
iii. Einstellen der Temperatur auf eine dritte Temperatur und Stoppen der Einleitung von Kohlendioxid vor dem Einleiten des Kohlenwasserstoffs,
wobei das Kohlendioxid in Schritt i) über den rezirkulierenden Stickstoffgasstrom eingeleitet wird und in Schritten ii) und iii) über den Dampf und optionalen rezirkulierenden Stickstoff.

4. Das Verfahren nach einem der Ansprüche 1 oder 2, **dadurch gekennzeichnet, dass** die fünfte Temperatur für eine Zeit von 30 Minuten bis 4 Stunden gehalten wird.

5. Das Verfahren nach Anspruch 1 oder 2, **dadurch gekennzeichnet, dass** die erste Temperatur in dem Bereich von 260°C bis 370°C ist.

6. Das Verfahren nach Anspruch 1 oder 2, **dadurch gekennzeichnet, dass** die zweite Temperatur in dem Bereich von 480°C bis 590°C ist.

7. Das Verfahren nach einem der Ansprüche 1 oder 2, **dadurch gekennzeichnet, dass** die dritte Temperatur in dem Bereich von 540°C bis 650°C ist.

8. Das Verfahren nach Anspruch 1 oder 2, **dadurch gekennzeichnet, dass** die vierte Temperatur in dem Bereich von 20°C bis 50°C ist.

9. Das Verfahren nach Anspruch 1 oder 2, **dadurch gekennzeichnet, dass** die fünfte Temperatur in dem Bereich von 540 °C bis 650 °C ist.

10. Das Verfahren nach einem der Ansprüche 1 bis 9, **dadurch gekennzeichnet, dass** der Katalysator ein Eisenoxid basierter Dehydrierungskatalysator ist umfassend 40 bis 90 Gew.-% Eisen und 5 bis 30 Gew.-% Kalium.

11. Das Verfahren nach einem der Ansprüche 1 bis 10, **dadurch gekennzeichnet, dass** der Kohlenwasserstoff ausgewählt ist aus der Gruppe von Alkylbenzolen wie Ethylbenzol, Methylethylbenzol, Diethylbenzole und Alkanen wie Propan, Butan und lineare Alkane von C9 bis C15.

## Revendications

1. Procédé d'exploitation d'un réacteur de déshydrogénation pour la déshydrogénation d'hydrocarbures, dans lequel le réacteur de déshydrogénation comprend un catalyseur à base d'oxyde de fer stimulé au potassium, les hydrocarbures étant déshydrogénés en contact avec le catalyseur, **caractérisé en ce que** le dioxyde de carbone est introduit durant
a) le démarrage du réacteur,
b) l'arrêt du réacteur, et
c) les procédures de traitement à la vapeur d'eau,
**caractérisé en ce que**
- le procédé comprend les étapes suivantes durant le démarrage (a) :
i. le chauffage du réacteur à une première température au moyen d'un courant gazeux en recirculation comprenant de l'azote,
ii. le chauffage du réacteur à une deuxième température par introduction de vapeur d'eau, et éventuellement la poursuite de la recirculation d'azote conjointement avec la vapeur d'eau,
iii. éventuellement la recherche de fuites, et
iv. le chauffage du réacteur à une troisième température et l'arrêt de l'introduction de dioxyde de carbone avant l'introduction des hydrocarbures,
dans lesquelles le dioxyde de carbone est introduit dans l'étape i) via le courant gazeux azoté en recirculation et dans les étapes ii) et iv) via la vapeur d'eau et éventuellement l'azote en recirculation,
- le procédé comprend les étapes suivantes durant l'arrêt (b) :
i. l'arrêt de l'introduction d'hydrocarbures dans le réacteur, le réacteur étant à une troisième température,
ii. le refroidissement du réacteur à une première température par utilisation d'un gaz réfrigérant comprenant de la vapeur d'eau et éventuellement addition d'azote et recirculation de l'azote conjointement avec la vapeur d'eau,
iii. le refroidissement du réacteur à une quatrième température par utilisation d'un gaz réfrigérant en recirculation comprenant de l'azote,
dans lesquelles le dioxyde de carbone est introduit dans l'étape ii) via le gaz réfrigérant comprenant de la vapeur d'eau et éventuellement de l'azote en recirculation et dans l'étape iii) via le gaz réfrigérant comprenant de l'azote,
- le procédé comprend les étapes suivantes durant les procédures de traitement à la vapeur d'eau (c) en partant d'un fonctionnement normal :
i. l'arrêt de l'introduction des hydrocarbures dans le réacteur, le réacteur étant à une troisième température,
ii. l'ajustement du réacteur et son maintien à une cinquième température avec de la vapeur d'eau et éventuellement l'addition d'azote et la recirculation de l'azote,
iii. l'ajustement de la température à une troisième température et l'arrêt de l'introduction de dioxyde de carbone avant l'introduction des hydrocarbures,
dans lesquelles le dioxyde de carbone est introduit dans les étapes ii) et iii) via la vapeur d'eau et éventuellement l'azote en recirculation,
dans lequel la concentration de dioxyde de carbone dans la vapeur d'eau est de 0,1 à 20 % en volume et la concentration de dioxyde de carbone dans le gaz azoté est de 2 à 50 % en volume.

2. Procédé selon la revendication 1, **caractérisé en ce que** la recirculation du dioxyde de carbone est effectuée.

3. Procédé selon la revendication 1 ou 2, **caractérisé en ce que** les procédures de traitement à la vapeur d'eau sont incorporées dans un démarrage comprenant les étapes suivantes :
i. le chauffage du réacteur à une première température au moyen d'un courant gazeux en recirculation comprenant de l'azote,
ii. le chauffage et le maintien à une cinquième température par introduction de vapeur d'eau et éventuellement la recirculation de l'azote,
iii. l'ajustement de la température à une troisième température et l'arrêt de l'introduction de dioxyde de carbone avant l'introduction des hydrocarbures,
dans lequel le dioxyde de carbone est introduit dans l'étape i) via le courant gazeux azoté en recirculation et dans les étapes ii) et iv) via la vapeur d'eau et éventuellement l'azote en recirculation.

4. Procédé selon l'une des revendications 1 et 2, **caractérisé en ce que** la cinquième température est maintenue pendant un temps de 30 minutes à 4 heures.

5. Procédé selon la revendication 1 ou 2, **caractérisé en ce que** la première température est située dans la plage allant de 260°C à 370°C.

6. Procédé selon la revendication 1 ou 2, **caractérisé en ce que** la deuxième température est située dans la plage allant de 480°C à 590°C.

7. Procédé selon la revendication 1 ou 2, **caractérisé en ce que** la troisième température est située dans la plage allant de 540°C à 650°C.

8. Procédé selon la revendication 1 ou 2, **caractérisé en ce que** la quatrième température est située dans la plage allant de 20°C à 50°C.

9. Procédé selon la revendication 1 ou 2, **caractérisé en ce que** la cinquième température est située dans la plage allant de 540°C à 650°C.

10. Procédé selon l'une quelconque des revendications 1 à 9, **caractérisé en ce que** le catalyseur est un catalyseur de déshydrogénation à base de fer comprenant de 40 à 90 % en poids de fer et de 5 à 30 % en poids de potassium.

11. Procédé selon l'une quelconque des revendications 1 à 10, **caractérisé en ce que** les hydrocarbures sont choisis dans le groupe des alkylbenzènes tels que l'éthylbenzène, les méthyléthylbenzènes, le diéthylbenzène, et des alcanes tels que le propane, le butane et les alcanes linéaires en C9 à C15.
